# EUROPEAN PATENT SPECIFICATION

(11) **EP 3 775 170 B1**
(45) Date of publication and mention of the grant of the patent: **30.07.2025**
(21) Application number: 19784485.5
(22) Date of filing: 19.03.2019
(51) Int. Cl.: A01K 67/0271, C12N 5/09, A61K 49/00, G01N 33/50

(54) **METHOD FOR OBTAINING A MOUSE MODEL FROM CONDITIONALLY REPROGRAMMED CELLS AND USE OF THE MOUSE MODEL FOR SCREENING ANTI-TUMOR DRUGS**
VERFAHREN ZUR GEWINNUNG EINES MAUSMODELLS AUS BEDINGT REPROGRAMMIERTEN ZELLEN UND VERWENDUNG DES MAUSMODELLS ZUM SCREENING VON ANTITUMORMEDIKAMENTEN
PROCÉDÉ D'OBTENTION D'UN MODÈLE MURIN À PARTIR DE CELLULES REPROGRAMMÉES CONDITIONNELLEMENT ET UTILISATION DU MODÈLE MURIN POUR LE CRIBLAGE DE MÉDICAMENTS ANTITUMORAUX

(30) Priority: 13.04.2018 WO PCT/CN2018/083110
(43) Date of publication of application: 17.02.2021
(73) Proprietor: Shanghai Lide Biotech Co., Ltd., Shanghai 201203 (CN)
(72) Inventor: WEN, Danyi, Shanghai 201203 (US)
(74) Representative: Fabry, Bernd
(86) International application number: PCT/CN2019/078703
(87) International publication number: WO 2019/196606

(56) References cited:
- WO-A1-03/101187
- WO-A1-2008/133359
- WO-A1-2014/092457
- CN-A- 107 541 494
- CN-A- 107 541 496
- CN-B- 103 898 056
- TIMOFEEVA OLGA A. ET AL: "Conditionally reprogrammed normal and primary tumor prostate epithelial cells: a novel patient-derived cell model for studies of human prostate cancer", ONCOTARGET, vol. 8, no. 14, 4 April 2017 (2017-04-04), pages 22741 - 22758, XP055796886, Retrieved from the Internet <URL:https://www.ncbi.nlm.nih.gov/pmc/articles/PMC5410259/pdf/oncotarget-08-22741.pdf> DOI: 10.18632/oncotarget.13937
- XUEFENG LIU ET AL: "ROCK Inhibitor and Feeder Cells Induce the Conditional Reprogramming of Epithelial Cells", THE AMERICAN JOURNAL OF PATHOLOGY, vol. 180, no. 2, 1 February 2012 (2012-02-01), pages 599 - 607, XP055131196, ISSN: 0002-9440, DOI: 10.1016/j.ajpath.2011.10.036
- LEE KEYONG HO ET AL: "Correlative Effect betw een in vivo H ollow Fiber A ssay and X enografts A ssay in Drug Screening INTRODUCTION", CANCER RES TREAT, vol. 37, 1 January 2005 (2005-01-01), pages 196 - 200, XP055872998, Retrieved from the Internet <URL:https://www.ncbi.nlm.nih.gov/pmc/articles/PMC2785405/pdf/crt-37-196.pdf> DOI: 10.4143/crt.2005.37.3.196
- LIU XUEFENG ET AL: "Conditional reprogramming and long-term expansion of normal and tumor cells from human biospecimens", NATURE PROTOCOLS, NATURE PUBLISHING GROUP, GB, vol. 12, no. 2, 26 January 2017 (2017-01-26), pages 439 - 451, XP037547631, ISSN: 1754-2189, [retrieved on 20170126], DOI: 10.1038/NPROT.2016.174
- RAKHI PAL ET AL: "Phenotypic and functional comparison of optimum culture conditions for upscaling of bone marrow-derived mesenchymal stem cells", JOURNAL OF TISSUE ENGINEERING AND REGENERATIVE MEDICINE, vol. 3, no. 3, 1 March 2009 (2009-03-01), pages 163 - 174, XP055173547, ISSN: 1932-6254, DOI: 10.1002/term.143

## Description

The present invention relates to a composition for propagating a primary tumor cell obtained from a tumor biopsy sample of a patient under both *in vitro and in vivo* conditions, a method for propagating the obtained primary tumor cell in the composition, a method for obtaining an animal model for screening the efficacy of an anti-tumor drug using the animal model.

### Background

Conditional reprogramming (CR) is a cell culture technique that can be used to rapidly and efficiently establish patient-derived cell cultures from both normal and diseased cells, including tumor cells. The greatest advantage of CR is its rapid and efficient expansion of cell cultures from patient tissue samples. This allows researchers to screen tumors for sensitivity to anticancer drugs or immunotherapies quickly enough to provide the information for clinical use.

The article "Conditionally reprogrammed normal and primary tumor prostate epithelial cells: a novel patient-derived cell model for studies of human prostate cancer" (Olga A. Timofeeva et al., Oncotarget. 2017 Apr 4; 8(14): 22741-22758.) concerns re-progammed tumor prostate epithelial cells used as a cell model for studying human prostate cancer. More particular, it discloses a method involving the step of cultivating said cells in F-medium consisting of 25% Ham's F-12 nutrient mix and 75% complete DMEM, supplemented with 25ng/ml hydrocortisone, 5 mg/ml insulin, 0.1 nmol/ml cholera toxin, 250 ng/ml Fungizone, 0.125 ng/ml epidermal growth factor, 10 mg/ml gentamicin and 5 µmol/L ROCK inhibitor Y-27632.

The article "Rock Inhibitor and Feeder Cells Induce the Conditional Reprogramming of Epithelial Cells" (Xuefeng Liu et al., The American Journal of Pathology Volume 180, Issue 2, February 2012, Pages 599-607) demonstrate a method comprising the step of cultivating the cells in the presence of the ROCK inhibitor Y-27632 in order to produce conditionally reprogrammed primary tumor cells.

The patent CN 103898056 B discloses an improved method for the preparation of reprogrammed cancer cells. The method therein comprises culturing the cells in a medium comprising hydrocortisone, cholera toxin, Insulin and on growth arrested feeder cells.

Testing an anti-tumor drug in an animal model for its efficacy is important as such animal model is important for anti-tumor drug screening. Such an animal model will mimic the in vivo environment of patients, reflect the patient's response and thus is more effective. To establish an animal models a person skilled in the art usually need to obtain sufficiently large number of tumor cells. However, tumor samples obtained from patients may contain very small or even trace amount of tumor cells depending on how they are obtained. For example, a tumor sample obtained from needle biopsy may contain very small amount of tumor cells and thus very difficult for a person skilled in the art to use them to establish a desired animal model for screening anti-tumor drugs.

Tumor cell immortalization through conditional reprogrammmg 1s an invaluable tool to generate propagating tumor cells for cell-based diagnostics, drug sensitivity assay and bio-banking in vitro. However, how to effectively and successfully cultivate primary tumor cells obtained from patients, especially low or even trace amount of cell samples from e.g. a needle biopsy, is still a challenge.

### Summary of the Invention

To solve the above problem, the inventor has successfully conditionally reprogrammed primary tumor cells from a number of tumor types towards immortalization and use the conditionally reprogrammed tumor cells to establish an animal model for testing anti-tumor drugs. The primary tumor cells are obtained from surgery tissues, biopsy or needle biopsy samples. The conditionally reprogrammed primary tumor cells obtained in the present invention exhibited typical colonized growth, which is well maintained upon cryo-frozen. In some cases, the cells can be passaged for multiple times and become useful cell lines. Like primary tumor cells, conditional reprogrammed tumor cells are reliable to test drug sensitivity *in vitro.* The inventor has successfully implanted conditional reprogrammed tumor cells (CRC) into an animal model for testing drug efficacy, and screened anti-tumor drugs using the animal model. Also described herein is the use of conditional reprogrammed tumor cells in drug efficacy tests.

One aspect of the present invention provides a method for obtaining a mouse model for screening anti-tumor drugs, comprising
(1) cultivating a primary tumor cell obtained from a tumor biopsy sample of a patient in a composition comprising:
   a) 0.01-1.0 mg/L Hydrocortisone;
   b) 1.0 -10.0 mg/L insulin;
   c) 1.0-20.0 µg/L Cholera toxin;
   d) 2.0-50.0 mg/L Adenine;
   e) 1.0-30.0 µg/L EGF;
   f) 1.0-30.0 µmol/L Y- 27632;
   g) Pen/Strep;
   h) 2-20% FBS;
   i) F12 medium
   j) DMEM with high glucose, and optionally
   k) Non-Essential Amino Acids Solution;
   l) GlutaMAX Supplement; and
   m) a mouse embryonic fibroblast cell that has been treated with mitomycin C; and
(2) implanting the primary tumor cell obtained from step (1) into an animal, wherein the animal is a mouse. In embodiments, the composition in step (1) of the method of the present invention comprises:
   a) 0.3-0.5 mg/L, preferably 0.4 mg/L Hydrocortisone;
   b) 4-6 mg/L, preferably 5 mg/L insulin;
   c) 7-9µg/L, preferably 8.3 µg/L Cholera toxin;
   d) 20-30 mg/L, preferably 24.2 mg/L Adenine;
   e) 8-12µg/L, preferably 10 µg/L EGF;
   f) 8-12µmol/L, preferably 10 µmol/L Y- 27632;
   g) Pen/Strep;
   h) 8-12%, preferably 10% FBS;
   i) F12 medium
   j) DMEM with high glucose, and optionally
   k) Non-Essential Amino Acids Solution;
   l) GlutaMAX Supplement.

In embodiments, the tumor biopsy sample is a needle biopsy sample. In embodiments, the concentration of the primary tumor cells in the sample obtained from a needle biopsy is less than about 2*10⁶/mL, less than about 1.9*10⁶/mL, less than about 1.8*10⁶/mL, less than about 1.7*10⁶/mL, less than about 1.6*10⁶/mL, less than about 1.5*10⁶/mL, less than about 1.4 *10⁶/mL, less than about 1.3 *10⁶/mL, less than about 1.2 *10⁶/mL, less than about 1.1 *10⁶/mL, less than about 1.0 *10⁶/mL, less than about 0.9 *10⁶/mL, less than about 0.8 *10⁶/mL, less than about 0,7 *10⁶/mL, less than about 0.6 *10⁶/mL, less than about 0.5 *10⁶/mL, less than about 0.4 *10⁶/mL, less than about 0.3 *10⁶/mL, less than about 0.2 *10⁶/mL, less than about 0.1 *10⁶/mL or less, such as less than about 2 *10⁶/mL, less than about 1.7 *10⁶/mL, less than about 0.8 *10⁶/mL, less than about 0.64 *10⁶/mL, less than about 0.59 *10⁶/mL. In embodiments, the primary tumor cell is cultivated with a feeder cell in the composition of step (1), in particular the feeder cell is a mouse embryonic fibroblast (MEF) cell. In embodiments, the MEF cells have been treated with mitomycin C.

The animal in the present invention is a mouse, in particular an immunodeficient mouse, such as a nude mouse. In embodiments, the primary tumor cell obtained in step (1) was implanted in to the animal in a hollow fiber. In embodiments, the hollow fiber is made of modified polyvinylidene fluoride, more particularly the hollow fiber is made of modified polyvinylidene fluoride and has a cut-off value of 500,000 Dalton.

### Drawing

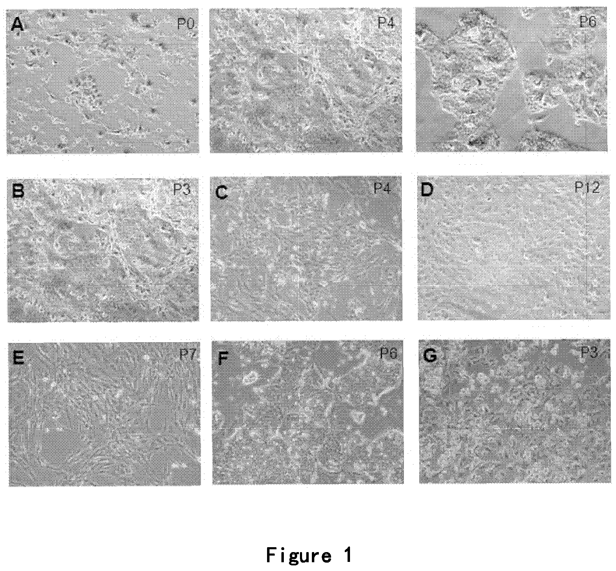

Figure **1** shows the morphology of conditionally reprogrammed cells in growing colonies from various tumor samples. **A,** PO, P4 and P6 passages of conditionally reprogrammed primary cells originated from surgical sample of a lung cancer patient; **B,** P3 conditionally reprogrammed cells originated from biopsy sample of a lung cancer patient; **C, P4** conditionally reprogrammed cells originated from a needle biopsy sample of an adenoid cystic carcinoma patient; D, P12 cell originated from a needle biopsy sample of a peritoneal mesothelioma patient; E, P7 cell originated from a surgical sample of a glioblastoma patient; F, P6 cell originated from a biopsy sample of a colonrectal cancer patient; G, P3 cell originated from a surgical sample of a gallbladder carcinoma patient.

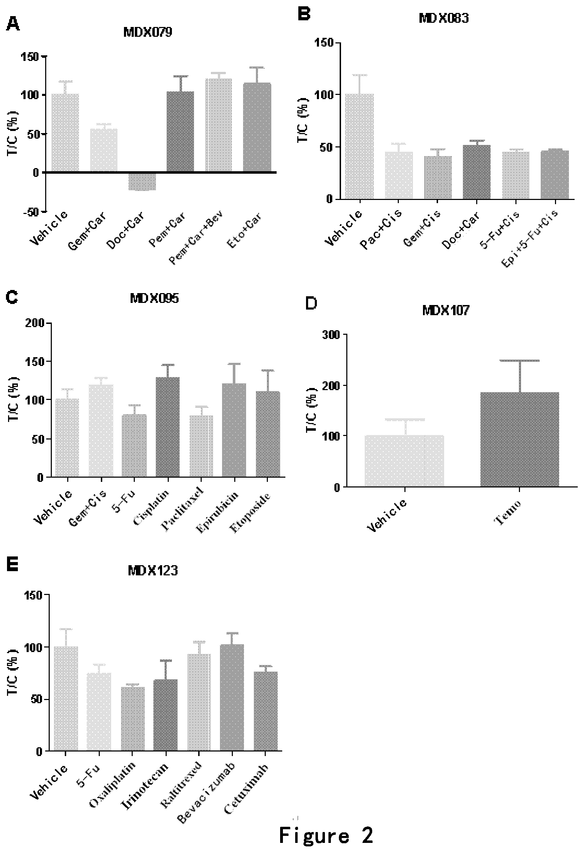

Figure 2 A, The results of anti-tumor efficacy of test drug in MDX079 Mini-PDX models; B, The results of anti-tumor efficacy of test drug in MDX083 Mini-PDX models; C, The results of anti-tumor efficacy of test drug in MDX095 Mini-PDX models; D, The results of anti-tumor efficacy of test drug in MDX107 Mini-PDX models; E, The results of anti-tumor efficacy of test drug in MDX123 Mini-PDX models.

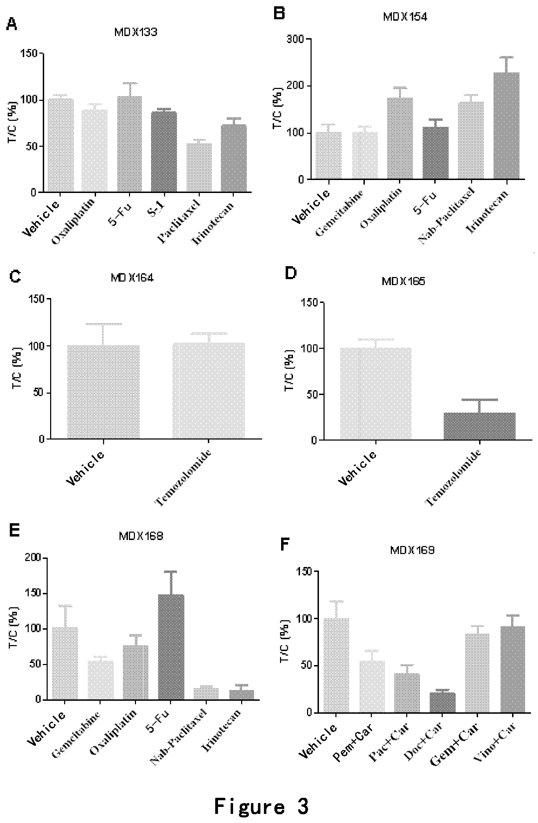

Figure 3 A, The results of anti-tumor efficacy of test drug in MDX133 Mini-PDX models; B, The results of anti-tumor efficacy of test drug in MDX154 Mini-PDX models; C, The results of anti-tumor efficacy of test drug in MDX164 Mini-PDX models; D, The results of anti-tumor efficacy of test drug in MDX165 Mini-PDX models; E, The results of anti-tumor efficacy of test drug in MDX168 Mini-PDX models. F, The results of anti-tumor efficacy of test drug in MDX169 Mini-PDX models; G, The results of anti-tumor efficacy of test drug in MDX174 Mini-PDX models; H, The results of anti-tumor efficacy of test drug in MDX186 Mini-PDX models; I, The results of anti-tumor efficacy of test drug in MDX189 Mini-PDX models; J, The results of anti-tumor efficacy of test drug in MDX203 Mini-PDX models.

### Detailed Description of the Invention

One aspect of the present invention provides a method for obtaining a mouse model for screening anti-tumor drugs, comprising (1) cultivating a primary tumor cell obtained from a tumor biopsy sample of a patient in a composition comprising:
a) 0.01-1.0 mg/L Hydrocortisone;
b) 1.0 -10.0 mg/L insulin;
c) 1.0-20.0 µg/L Cholera toxin;
d) 2.0-50.0 mg/L Adenine;
e) 1.0-30.0 µg/L EGF;
f) 1.0-30.0 µmol/L Y- 27632;
g) Pen/Strep;
h) 2-20% FBS;
i) F12 medium
j) DMEM with high glucose, and optionally
k) Non-Essential Amino Acids Solution;
l) GlutaMAX Supplement; and
m) a mouse embryonic fibroblast cell that has been treated with mitomycin C; and

(2) implanting the primary tumor cell obtained from step (1) into an animal, wherein the animal is a mouse. In embodiments, the tumor biopsy sample is a needle biopsy sample. In embodiments, the concentration of tumor cells in the sample obtained from the needle biopsy is less than about 2*10⁶/mL.

The concentration of hydrocortisone in the composition of step (1) can be 0.01-1.0 mg/L, in particular about 0.05-1.0 mg/L, about 0.1-1.0 mg/L, about 0.1-0.9 mg/L, about 0.2-0.8 mg/L, about 0.2-0.7 mg/L, about 0.25-0.65mg/L, about 0.25-0.6mg/L, about 0.3-0.5mg/L, about 0.35-0.55mg/L, about 0.35-0.4mg/L, about 0.4-0.45mg/L, about 0.35-0.45mg/L, about 0.45-0.55mg/L, about 0.05 mg/L, about 0.1 mg/L, about 0.2 mg/L, about 0.3 mg/L, about 0.31 mg/L, about 0.32 mg/L, about 0.33 mg/L, about 0.34 mg/L, about 0.35 mg/L, about 0.36 mg/L, about 0.37 mg/L, about 0.38 mg/L, about 0.39 mg/L, about 0.4 mg/L, about 0.41 mg/L, about 0.42 mg/L, about 0.43 mg/L, about 0.44 mg/L, about 0.45 mg/L, about 0.46 mg/L, about 0.47 mg/L, about 0.48 mg/L, about 0.49 mg/L, about 0.5 mg/L, about 0.6 mg/L, about 0.7 mg/L, about 0.8 mg/L, about 0.9 mg/L, about 1.0 mg/L.

The concentration of insulin in the composition of step (1) can be 1.0-10 mg/L, in particular about 1-10 mg/L, about 2-9 mg/L, about 3-8 mg/L, about 3-7 mg/L, about 4.5-6.5 mg/L, about 4-6 mg/L, about 1 mg/L, about 2 mg/L, about 3 mg/L, about 4 mg/L, about 5 mg/L, about 6 mg/L, about 7 mg/L, about 8 mg/L, about 9 mg/L, about 10 mg/L.

The concentration of cholera toxin in the composition of step (1) can be 1-20 µg/L, in particular about 2-20 µg/L, about 3-18 µg/L, about 4-15 µg/L, about 5-10 µg/L, about 6-10 µg/L, about 7-9 µg/L, about 7.5-8.5 µg/L, about 1 µg/L, about 2 µg/L, about 3 µg/L, about 4 µg/L, about 5 µg/L, about 6 µg/L, about 7 µg/L, about 8 µg/L, about 8.1 µg/L, about 8.2 µg/L, about 8.3 µg/L, about 8.4 µg/L, about 8.5 µg/L, about 8.6 µg/L, about 8.7 µg/L, about 8.8 µg/L, about 8.9 µg/L, about 9 µg/L, about 10 µg/L, about 11 µg/L, about 12 µg/L, about 13 µg/L, about 14 µg/L, about 15 µg/L, about 16 µg/L, about 17 µg/L, about 18 µg/L, about 19 µg/L, about 20 µg/L.

The concentration of adenine in the composition of step (1) can be 2-50 mg/L, in particular about 5-40 mg/L, about 10-30 mg/L, about 15-30 mg/L, about 20-30 mg/L, about 22-27 mg/L, about 23-26 mg/L, about 24-25 mg/L, about 2 mg/L, about 6 mg/L, about 8 mg/L, about 10 mg/L, about 12 mg/L, about 14 mg/L, about 16 mg/L, about 18 mg/L, about 20 mg/L, about 21 mg/L, about 22 mg/L, about 23 mg/L, about 24 mg/L, about 24.1 mg/L, about 24.2 mg/L, about 24.3 mg/L, about 24.4 mg/L, about 24.5 mg/L, about 24.6 mg/L, about 24.7 mg/L, about 24.8 mg/L, about 24.9 mg/L, about 25 mg/L, about 26 mg/L, about 27 mg/L, about 28 mg/L, about 29 mg/L, about 30 mg/L, about 35 mg/L, about 40 mg/L.

The concentration of EGF in the composition of step (1) can be 1-30 µg/L, in particular about 2-20 µg/L, about 4-18 µg/L, about 6-16 µg/L, about 8-12 µg/L, about 9-11 µg/L, about 9.5-10.5 µg/L, about 2 µg/L, about 4 µg/L, about 6 µg/L, about 7 µg/L, about 8 µg/L, about 9 µg/L about 10 µg/L, about 11 µg/L, about 12 µg/L, about 14 µg/L, about 16 µg/L, about 18 µg/L, about 20 µg/L, about 25 µg/L, about 30 µg/L.

The concentration of Y-27632 in the composition of step (1) can be 1-30 µmol/L, in particular about 2-20µmol/L, about 4-18µmol/L, about 6-16µmol/L, about 8-12µmol/L, about 9-llµmol/L, about 9.5-10.5µmol/L, about 2µmol/L, about 4µmol/L, about 6µmol/L, about 7µmol/L, about 8µmol/L, about 9µmol/L, about lOµmol/L, about llµmol/L, about 12µmol/L, about 14µmol/L, about 16µmol/L, about 18µmol/L, about 20µmol/L, about 25µmol/L, about 30µmol/L.

The concentration of FBS the composition of step (1) can be 2-20%, in particular about 4-15%, about 5-18%, about 6-16%, about 7-14%, about 8-12%, about 9-11%, about 9.5-10.5%, about 2%, about 4 %, about 6%, about 7%, about 8%, about 9%, about 10%, about 11%, about 12%, about 14%, about 16 %, about 18%, about 20%.

In embodiments, the present invention provides a method for obtaining an animal model for screening anti-tumor drugs, comprising (1) cultivating a primary tumor cell obtained from a tumor biopsy sample of a patient in a composition comprising:
a) 0.3-0.5 mg/L, preferably 0.4 mg/L Hydrocortisone;
b) 4-6 mg/L, preferably 5 mg/L insulin;
c) 7-9µg/L, preferably 8.3 µg/L Cholera toxin;
d) 20-30 mg/L, preferably 24.2 mg/L Adenine;
e) 8-12µg/L, preferably 10 µg/L EGF;
f) 8-12µmol/L, preferably 10 µmol/L Y- 27632;
g) Pen/Strep;
h) 8-12%, preferably 10% FBS;
i) F12 medium
j) DMEM with high glucose, and optionally
k) Non-Essential Amino Acids Solution;
l) GlutaMAX Supplement; and
m) a mouse embryonic fibroblast cell that has been treated with mitomycin C; and
(2) implanting the primary tumor cell obtained from step (1) into an animal, wherein the animal is a mouse. In embodiments, the tumor biopsy sample is a needle biopsy sample. In embodiments, the concentration of the primary tumor cells in the sample obtained from a needle biopsy is less than about 2*10⁶/mL,
less than about 1.9*10⁶/mL, less than about 1.8*10⁶/mL, less than about 1.7*10⁶/mL, less than about 1.6*10⁶/mL, less than about 1.5*10⁶/mL, less than about 1.4 *10⁶/mL, less than about 1.3 *10⁶/mL, less than about 1.2 *10⁶/mL, less than about 1.1 *10⁶/mL, less than about 1.0 *10⁶/mL, less than about 0.9 *10⁶/mL, less than about 0.8 *10⁶/mL, less than about 0,7 *10⁶/mL, less than about 0.6 *10⁶/mL, less than about 0.5 *10⁶/mL, less than about 0.4 *10⁶/mL, less than about 0.3 *10⁶/mL, less than about 0.2 *10⁶/mL, less than about 0.1 *10⁶/mL or less, such as less than about 2 *10⁶/mL, less than about 1.7 *10⁶/mL, less than about 0.8 *10⁶/mL, less than about 0.64 *10⁶/mL, less than about 0.59 *10⁶/mL. In embodiments, the primary tumor cell is cultivated with a feeder cell in the composition of step (1), in particular the feeder cell is a mouse embryonic fibroblast (MEF) cell. In embodiments, the MEF cells have been treated with mitomycin C.

In embodiments, the animal in the method of the present invention is a mouse, in particular an immunodeficient mouse, such as a nude mouse. In embodiments, the primary tumor cell obtained in step (1) was implanted in to the animal in a hollow fiber. In embodiments, the hollow fiber is made of modified polyvinylidene fluoride, more particularly the hollow fiber is made of modified polyvinylidene fluoride and has a cut-off value of 500,000 Dalton.

The tumor cells can be obtained from a variety of tumor types, including but not limited to tumors in the digestive tract (such as the stomach, intestines, duodenum, colon, pancreas, etc.), the breast, lung, liver, and endocrine glands (such as the adrenal gland, parathyroid gland, pituitary, testis, ovaries, and thymus, thyroid gland), urinary and reproductive systems (such as kidney, bladder, ovary, testis, prostate, etc.), skeletal muscle system (such as bone, smooth muscle, striated muscle, etc.), skin, and so on. For example, the tumor cells can be derived from gastric cancer, biopsy specimen of colorectal cancer and lung cancer.

The conditionally reprogrammed primary tumor cells can be implanted into the animal via a syringe or other methods or devices known in the art. In embodiments, the conditionally reprogrammed primary tumor cells are implanted into an animal by a method for producing a PDX model for tumor growth in vivo. See e.g. "Melanoma patient-derived xenografts accurately model the disease and develop fast enough to guide treatment decisions" , Oncotarget, Vol. 5, No. 20, Berglind 0. Einarsdottir et.al., published on Sep 8 2014, " Personalizing Cancer Treatment in the Age of Global Genomic Analyses: PALB2 Gene Mutations and the Response to DNA Damaging Agents in Pancreatic Cancer", Molecular Cancer Therapies, published on Dec 6 2010; DOI: 10.1158/1535-7163.MCT-10-0893, Maria C. Villarroel. In embodiments, the conditionally reprogrammed primary tumor cells were transferred into a hollow-fiber tube, which is then implanted into an animal. The hollow fiber can be made of modified polyvinylidene fluoride and has a cut-off value of 500,000 Dalton. In the present invention, a mini-PDX device in the refers to a hollow fiber can be made of modified polyvinylidene fluoride and has a cut-off value of 500,000 Dalton, which may contain the primary tumor cells obtained from a patient and be implanted into a candidate animal, e.g. implanted into a candidate animal subcutaneously. In the present invention, a mini-PDX model refers to an animal that has been implanted with the mini-PDX device of the present invention. The mini-PDX animal model can be used in the methods/tests described in the present invention.

### Examples

The present invention will be better described below with reference to the accompanymg drawings.

### Conditional reprograming medium

Medium contains the following ingredients:
- 375 mL F12 medium (commercially available), 125 mL DMEM with high glucose (commercially available), 25 mL 10% FBS (commercially available), 5 mL hydrocortisone, 5 mL insulin, 5 mL cholera toxin, 5 mL adenine, 5 mL Pen/Strep (commercially available), 5 mL EGF, lmL Y- 27632.
- Optional ingredients: Non-Essential Amino Acids Solution (commercially available); and GlutaMAX Supplement (commercially available).

### Preparation:

The following ingredients are prepared:
- Hydrocortisone: dissolve 25 mg commercially available Hydrocortisone in 5 mL cold 100% ethanol to make a 5 mg/mL solution. Add 0.8 mL of said 5 mg/mL solution to 100 mL Hanks' Balanced Salt Solution (HBSS)containing 5% fetal bovine serum (FBS). Filter sterilize and store at -20°C in 10 mL aliquots. The final concentration of hydrocortisone is 0.4mg/L.
- Cholera toxin: add 1.2 mL of sterile water to a vial containingl-mg cholera toxin (commercially available), obtaining a 10 µM solution. Dilute 50 µL of said 10 µM solution into 50 mL HBSS containing 0.1% bovine serum albumin. Filter sterilize and store at 4°C in 10 mL aliquots. The final concentration of cholera toxin is 8.3 µg/L.
- Insulin: dissolve 12.5 mg commercially available insulin in 25 mL of 0.005 M HCL. Filter sterilize with a syringe filter pre-wet with FBS. The final concentration of insulin is 5 mg/L.
- Adenine: dissolve 121 mg commercially available adenine in 50 mL of 0.05 M HCl by stirring for 1 h. Filter sterilize and store at -20°C in 10 mL aliquots. The final concentration of adenine is 24.2 mg/L.
- Epidermal growth factor (EGF): dissolve 100 µg commercially available EGF in 10 mL sterile water. Add 90 mL HBSS containing 0.1% bovine serum albumin. Filter sterilize and store at -20°C in 10 mL aliquots. The final concentration of EGF is 10 µg/L.
- Y- 27632; 10 µmol in DMSO. Commercially available.

The medium was then properly kept in a cool place for use.

### Feeder cell preparation:

MEF (mouse embryonic fibroblast) cells were isolated from e13.5 embryos of C57 mouse and were grown in DMEM supplemented with 10% FBS. Isolated MEF cells in 3-5 passages were treated with mitomycin C (I0µg/ml) for 2h and were washed with PBS. The treated MEF were harvested and cryopreserved as feeder cell.

### Example 1

### 1. Materials

### 1.1 Tumors for conditional reprograming

Conditional reprograming cells from MDX079, MDX083, MDX095, MDX107, MDX123 were collected separately. MDX079 originated from a female lung cancer patient, MDX083 model originated from a 27-year old male adenoid cystic carcinoma patient, MDX095 model originated from a 54-year old male peritoneal malignant tnesoshelioma patient, MDX107 model originated from a 54-year old male glioblastoma patient, MDX123 model originated from a 53-year old male colonrectal cancer patient.

### 1.2 Animals

1.2.1 Balb/c nude, female, were purchased from Shanghai Laboratory Animal Center (Lingchang, Shanghai, China, SCXK(SH)2013-0018).
   Age: 6-8 weeks
   Sex: female
   Body weight: 18-22 g
1.2.2 Housing condition
   The mice were kept in SPF room at constant temperature and humidity with 3 animals in each cage.
      - Temperature: 20- 26 °C.
      - Humidity: 40-70 %.
      - Light cycle: 12 hours light and 12 hours dark.
   Cages: Made of polycarbonate. The size is 325 mm x 210 mm x 180 mm. The bedding material is corn cob, which is changed twice per week.
   Diet: Animals had free access to irradiation sterilized dry granule food during the entire study period.
   Water: Animals had free access to sterile drinking water.
   Cage identification: the identification labels for each cage contain the following information: number of animals, sex, strain, date received, treatment, study number, group number, and the starting date of the treatment. Animal identification: Animals were marked by ear coding.

### 1.3 Equipments

Reverse microscope DMIL, LEICA. Balance ALC-310.3, Acclulab. Microbalance BSA224S, Sartorius. Centrifuge 5810R, Eppendorf.

Mini-PDX device: a hollow fiber made of modified polyvinylidene fluoride which has a cut-off value of 500,000 Dalton, internal diameter 1-2 mm. Cut into desired length.

### 2. Procedure and methods

### 2.1 Cell Culture

2.1.1
   Wash the tumor tissue sample with buffer solution and remove away non-tumor tissue and necrotic tumor tissue in biosafety cabinet. Cut the tumor into 1-3 mm³ fragments, suspend pellet with IX collagenase solution and incubate in 37°C for 1-2 hours. Collect single cells through 70uM strainer, count cell numbers to calculate the tumor cell concentration (See 9. Tumor cell concentration obtained from surgery and biopsy/needle biopsy). Cultivate the cells with the conditional reprograming medium, and after tumor cells proliferation, harvest the conditional reprograming cells.
2.1.2
   Suspend tumor tissues obtained from biopsy/needle biopsy in IX collagenase solution and incubate in 37°C for 1-2 hours. Collect single cells through 70uM strainer, count cell numbers to calculate the tumor cell concentration (See 9. Tumor cell concentration obtained from surgery and biopsy/needle biopsy). Cultivate the cells with the conditional reprograming medium and after tumor cells proliferation harvest the conditional reprograming cells.

### 2.2 Mini-PDX device Inoculation

The cells suspension was filled into Mini-PDX device and the devices were inoculated subcutaneously into both flanks of Nu/Nu-nude mice, to establish the Mini-PDX model. The inoculation day was defined as day 0. Mice were randomized in groups according to the bodyweights and the treatments were initiated at day 0. The test article administration and the mini-PDX device numbers in each study group are shown in the following experimental design tables.

**Table 1 MDX079 Groups and Treatment**

| **Group** | **N** | **Treatment** | **Dose (mg/kg)** | **Dosing Route** | **Schedule** |
|---|---|---|---|---|---|
| 1 | 5 | Vehicle | 0 | *i.p.* | qd*5 |
| 2 | 6 | Gemcitabin e+Carbopla | 60+50 | *i.p.* | q4d*2 +qw |
| 3 | 6 | Docetaxel +Carboolati | 20+50 | *i.p.* | q4d*2 +qw |
| 4 | 6 | Pemetrexed + | 200+50 | *i.p.* | qd*5 +qw |
| 5 | 6 | Pemetrexed + Carboplatin | 200+50+10 | *i.p.* | qd*5 +qw+ q4d*2 |
| 6 | 6 | Etoposide+ Carboplatin | 20+50 | *i.p.* | q4d*2 +qw |

| | | | | | |
|---|---|---|---|---|---|
| Note: N: mini-PDX device number; i.p.: intraperitoneal injection; qd: Once every day; q4d: Once every 4 days; qw: Once every week; | | | | | |

**Table 2 MDX083 Groups and Treatment**

| **Group** | **N** | **Treatment** | **Dose (mg/kg)** | **Dosing Route** | **Schedule** |
|---|---|---|---|---|---|
| 1 | 6 | Vehicle | 0 | *i.p.* | qd*5 |
| 2 | 6 | Paclitaxel +Cisplatin | 20+5 | *i.p.* | q4d*2 +qw |
| 3 | 6 | Gemcitabine + Cisolatin | 60+5 | *i.p.* | q4d*2 +qw |
| 4 | 6 | Docetaxel+ Carboplatin | 20+50 | *i.p.* | q4d*2 +qw |
| 5 | 6 | 5-Fu +Cisplatin | 25+5 | *i.p.* | qd*5 +qw |
| 6 | 6 | Epirubicin+ 5-Fu+ | 5+25+5 | *i.p.* | qw+ qd*5+qw |

| | | | | | |
|---|---|---|---|---|---|
| Note: N: mini-PDX device number; i.p.: intraperitoneal injection; qd: Once every day; q4d: Once every 4 days; qw: Once every week; | | | | | |

**Table 3 MDX095 Groups and Treatment**

| **Group** | **N** | **Treatment** | **Dose (mg/kg)** | **Dosing Route** | **Schedule** |
|---|---|---|---|---|---|
| 1 | 6 | Vehicle | 0 | *i.p.* | qd*5 |
| 2 | 4 | Gemcitabine + Cisplatin | 60+5 | *i.p.* | q4d*2 +qw |
| 3 | 6 | 5-Fu | 25 | *i.p.* | qd*5 |
| 4 | 6 | Cisplatin | 5 | *i.p.* | q4d*2 |
| 5 | 6 | Paclitaxel | 20 | *i.p.* | q4d*2 |
| 6 | 6 | Epirubicin | 10 | *i.p.* | q4d*2 |
| 7 | 6 | Etoposide | 20 | *i.p.* | q4d*2 |

| | | | | | |
|---|---|---|---|---|---|
| Note: N: mini-PDX device number; i.p.: intraperitoneal injection; qd: Once every day; q4d: Once every 4 days; qw: Once every week; | | | | | |

**Table 4 MDX107 Groups and Treatment**

| **Group** | **N** | **Treatment** | **Dose (mg/kg)** | **Dosing Route** | **Schedule** |
|---|---|---|---|---|---|
| 1 | 6 | Vehicle | 0 | *p.a.* | qd*5 |
| 2 | 6 | Temozol omide | 50 | *p.a.* | qd*5 |

| | | | | | |
|---|---|---|---|---|---|
| Note: N: mini-PDX device number; p.o.: per os; qd: Once every day; | | | | | |

**Table 5 MDX123 Groups and Treatment**

| **Group** | **N** | **Treatment** | **Dose (mg/kg)** | **Dosing Route** | **Schedule** |
|---|---|---|---|---|---|
| 1 | 6 | Vehicle | 0 | *i.p.* | qd*5 |
| 2 | 6 | 5-Fu | 25 | *i.p.* | qd*5 |
| 3 | 6 | Oxaliplatin | 5 | *i.p.* | q4d*2 |
| 4 | 6 | Irinotecan | 50 | *i.p.* | q4d*2 |
| 5 | 6 | Raltitrexed | 30 | *i.p.* | q4d*2 |
| 6 | 6 | Bevacizumab | 10 | *i.p.* | q4d*2 |
| 7 | 6 | Cetuximab | 30 | *i.p.* | q4d*2 |

| | | | | | |
|---|---|---|---|---|---|
| Note: N: mini-PDX device number; i.p.: intraperitoneal injection; qd: Once every day; q4d: Once every 4 days; | | | | | |

### 2.3 Observation

The protocol and any amendment(s) or procedures involving the care and use of animals in this study were reviewed and approved by the Institutional Animal Care and Use Committee (IACUC) of Shanghai LIDE prior to conduct. During the study, the care and use of animals were conducted in accordance with the regulations of the Association for Assessment and Accreditation of Laboratory Animal Care (AAALAC). After inoculation, the animals were checked daily for morbidity and mortality. At the time of routine monitoring, the animals were checked for any effects of tumor growth and treatments on normal behavior such as mobility, food and water consumption, body weight gain/loss (body weight were measured twice weekly or every other day), eye/hair matting and any other abnormal effect. Death and observed clinical signs were recorded on the basis of the numbers of animals within each subset.

### 2.4 Endpoints

2.4.1 Body weight were measured every day, the treatment periods of these studies were 7 days, at the termination, all mice were euthanized and the Mini-PDX devices were removed for CTG (cell viability) assay. Tumor relative proliferation rate (%) is the indication of the anti-tumor activity.
2.4.2 When V cd7-V do > 0, Tumor relative proliferation rate(%) = (V rnr V do) / (V cdr V do) x100%; When V cd7-V do < 0, Tumor relative proliferation rate(%) = V Td7/ V cd7 x100% (V Td7: the cell viability on day 7 of treatment group; V cd7: the cell viability on day 7 of control group; Vdo: the cell viability on day 0).

### 3. Results (see Figure 2)

### 4. Summary and Discussion

In MDX079 Mini-PDX models, Docetaxel and Carboplatin combination treatment (T/C%=-21 %) could result in a significant decrease in tumor cell viability; Gemcitabine and Carboplatin combination treatment (T/C%=55%) also shown an anti-tumor activity; Pemetrexed and Carboplatin combination treatment (T/C%=103%) treatment, Pemetrexed, Carboplatin and Bevazulimab combination treatment (T/C%=119%) treatment, Etoposide and Carboplatin combination treatment (T/C%=114%) shown no anti-tumor activity. In MDX083 Mini-PDX models, all the treatment group include Paclitaxel and Cisplatin combination treatment (T/C%=44%), Gemcitabine and Cisplatin combination treatment (T/C%=41 %), Docetaxel and Carboplantin combination treatment (T/C%=50%), 5-Fu and Cisplatin combination treatment (T/C%=45%), Epirubicin, 5-Fu and Cisplatin combination treatment *(TIC%*=45%) could result in a decrease in tumor cell viability.

In MDX095 Mini-PDX models, 5-Fu treatment (T/C%=80%), Paclitaxel treatment (T/C%=79%) result in a little decrease in tumor cell viability; Gemcitabine and Cisplatin combination treatment (T/C%=119%) shown no anti-tumor activity, Cisplatin treatment (T/C%=128%), Epirubicin treatment (T/C%=120%), Etoposide treatment (T/C%=109%) shown no anti-tumor activity.

In MDX107 Mini-PDX models, Temozolomide treatment shown no anti-tumor activity. In MDX123 Mini-PDX models, 5-Fu treatment (T/C%=75%), Oxaliplatin treatment (T/C%=61 %), Irinotecan treatment (T/C%=68%), Bevazulimab treatment (T/C%=76%) could result in a decrease in tumor cell viability; Raltitrexed treatment (T/C%=92%), Bevazulimab treatment (T/C%=101 %) shown no anti-tumor activity.

### Example 2

### 5. Materials

### 5.1 Tumors for conditional reprograming

Conditional reprograming cells from MDX133, MDX154, MDX164, MDX165, MDX168, MDX169, MDX174, MDX186, MDX189, MDX203 were collected separately. MDX133 originated from a 45-year old male gastric cancer patient, MDX154 model originated from a female gallbladder carcinoma patient, MDX164 model originated from a 43-year old male glioblastoma patient, MDX165 model originated from a 64-year old male glioblastoma patient, MDX168 model originated from a 66-year old female gallbladder carcinoma patient, MDX169 model originated from a 52-year old male lung cancer patient, MDXI74 model originated from a 59-year old male lung cancer patient, MDX186 model originated from a 54-year old female pancreatic cancer patient, MDX189 model originated from a 44-year old male esophagus cancer patient, MDX203 model originated from a 61-year old male gallbladder carcinoma patient.

### 5.2 Animals

5.2.1 Balb/c nude, female, were purchased from Shanghai Laboratory Animal Center (Lingchang, Shanghai, China, SCXK(SH)2013-0018).
   Age: 6-8 weeks
   Sex: female
   Body weight: 18-22 g
5.2.2 Housing condition
   The mice were kept in SPF room at constant temperature and humidity with 3 animals in each cage.
   - Temperature: 20- 26 °C.
   - Humidity: 40-70 %.
   - Light cycle: 12 hours light and 12 hours dark.

Cages: Made of polycarbonate. The size is 325 mm x 210 mm x 180 mm. The bedding material is corn cob, which is changed twice per week.

Diet: Animals had free access to irradiation sterilized dry granule food during the entire study period.

Water: Animals had free access to sterile drinking water.

Cage identification: the identification labels for each cage contain the following information: number of animals, sex, strain, date received, treatment, study number, group number, and the starting date of the treatment. Animal identification: Animals were marked by ear coding.

### 5.3 Equipments

Reverse microscope DMIL, LEICA. Balance ALC-310.3, Acclulab. Microbalance BSA224S, Sartorius. Centrifuge 5810R, Eppendorf.

Mini-PDX device: a hollow fiber made of modified polyvinylidene fluoride which has a cut-off value of 500,000 Dalton, internal diameter 1-2 mm. Cut into desired length.

### 6. Procedure and methods

### 6.1 Cell Culture

6.1.1
   Wash the tumor tissue sample with buffer solution and remove away non-tumor tissue and necrotic tumor tissue in biosafety cabinet. Cut the tumor into 1-3 mm3 fragments, suspend pellet with IX collagenase solution and incubate in 37°C for 1-2 hours. Collect single cells through 70uM strainer, count cell numbers to calculate tumor cell concentration (See 9. Tumor cell concentration obtained from surgery and biopsy/needle biopsy). Cultivate the cells with conditional reprograming medium, after tumor cells proliferation, and harvest the conditional reprograming cells.
6.1.2
   Suspend tumor tissues obtained from biopsy/needle biopsy in IX collagenase solution and incubate in 37°C for 1-2 hours. Collect single cells through 70uM strainer, count cell numbers to calculate tumor cell concentration (See 9. Tumor cell concentration obtained from surgery and biopsy/needle biopsy). Cultivate the cells with conditional reprograming medium, after tumor cells proliferation, and harvest the conditional reprograming cells.

### 6.2 Mini-PDX device Inoculation

The cells suspension was filled into Mini-PDX device and the devices were inoculated subcutaneously into both flanks of Nu/Nu-nude mice, to establish the Mini-PDX model. The inoculation day was defined as day 0. Mice were randomized in groups according to the bodyweights and the treatments were initiated at day 0. The test article administration and the mini-PDX device numbers in each study group are shown in the following experimental design tables.

**Table 6 MDX133 Groups and Treatment**

| **Group** | **N** | **Treatment** | **Dose (mg/kg)** | **Dosing Route** | **Schedule** |
|---|---|---|---|---|---|
| 1 | 6 | Vehicle | 0 | *i.p.* | qd*5 |
| 2 | 6 | Oxaliplatin | 5 | *i.p.* | q4d*2 |
| 3 | 6 | 5-Fu | 25 | *i.p.* | qd*5 |
| 4 | 6 | S-1 | 10 | *p.a.* | qd*5 |
| 5 | 6 | Paclitaxel | 20 | *i.p.* | q4d*2 |
| 6 | 6 | Irinotecan | 50 | *i.p.* | q4d*2 |

| | | | | | |
|---|---|---|---|---|---|
| Note: N: mini-PDX device number; i.p.: intraperitoneal injection; p.o.: per os; qd: Once every day; q4d: Once every 4 days; | | | | | |

**Table 7 MDX154 Groups and Treatment**

| **Group** | **N** | **Treatment** | **Dose (mg/kg)** | **Dosing Route** | **Schedule** |
|---|---|---|---|---|---|
| 1 | 6 | Vehicle | 0 | *i.p.* | qd*5 |
| 2 | 6 | Gemcitabine | 60 | *i.p.* | q4d*2 |
| 3 | 6 | Oxaliplatin | 5 | *i.p.* | q4d*2 |
| 4 | 6 | 5-Fu | 25 | *i.p.* | qd*5 |
| 5 | 6 | Nab-Paclitax el | 20 | *i. v.* | qd*5 |
| 6 | 6 | Irinotecan | 50 | *i.p.* | q4d*2 |

| | | | | | |
|---|---|---|---|---|---|
| Note: N: mini-PDX device number; i.p.: intraperitoneal injection; i.v.: intravenous injection; qd: Once every day; q4d: Once every 4 days; | | | | | |

**Table 8 MDX164 Groups and Treatment**

| **Group** | **N** | **Treatment** | **Dose (mg/kg)** | **Dosing Route** | **Schedule** |
|---|---|---|---|---|---|
| 1 | 6 | Vehicle | 0 | *p.a.* | qd*5 |
| 2 | 6 | Temozolomi de | 50 | *p.a.* | qd*5 |

| | | | | | |
|---|---|---|---|---|---|
| Note: N: mini-PDX device number; p.o.: per os; qd: Once every day; | | | | | |

**Table 9 MDX165 Groups and Treatment**

| **Group** | **N** | **Treatment** | **Dose** (mg/kg) | **Dosing Route** | **Schedule** |
|---|---|---|---|---|---|
| 1 | 6 | Vehicle | 0 | *p.a.* | qd*5 |
| 2 | 6 | Temozol omide | 50 | *p.a.* | qd*5 |

| | | | | | |
|---|---|---|---|---|---|
| Note: N: mini-PDX device number; p.o.: per os; qd: Once every day; | | | | | |

**Table 10 MDX168 Groups and Treatment**

| **Group** | **N** | **Treatment** | **Dose (mg/kg)** | **Dosing Route** | **Schedule** |
|---|---|---|---|---|---|
| 1 | 6 | Vehicle | 0 | *i.p.* | qd*5 |
| 2 | 6 | Gemcitabine | 60 | *i.p.* | q4d*2 |
| 3 | 5 | Oxaliplatin | 5 | *i.p.* | q4d*2 |
| 4 | 5 | 5-Fu | 25 | *i.p.* | qd*5 |
| 5 | 5 | Nab-Paclitax el | 20 | *i.v*. | qd*5 |
| 6 | 6 | Irinotecan | 50 | *i.p.* | q4d*2 |

| | | | | | |
|---|---|---|---|---|---|
| Note: N: mini-PDX device number; i.p.: intraperitoneal injection; i.v.: intravenous injection; qd: Once every day; q4d: Once every 4 days; | | | | | |

**Table 11 MDX169 Groups and Treatment**

| **Group** | **N** | **Treatment** | **Dose (mg/kg)** | **Dosing Route** | **Schedule** |
|---|---|---|---|---|---|
| 1 | 6 | Vehicle | 0 | *i.p.* | qd*5 |
| 2 | 6 | Pemetrexed + Carboplatin | 200+50 | *i.p.* | qd*5 +qw |
| 3 | 6 | Paclitaxel + Carboolatin | 20+50 | *i.p.* | q4d*2 +qw |
| 4 | 6 | Docetaxel +Carboplati | 20+50 | *i.p.* | q4d*2 +qw |
| 5 | 6 | Gemcitabin e+Carbopla | 60+50 | *i.p.* | q4d*2 +qw |
| 6 | 6 | Vinorelbine +Carboplati | 5+50 | *i.p.* | q4d*2+q w |

| | | | | | |
|---|---|---|---|---|---|
| Note: N: mini-PDX device number; i.p.: intraperitoneal injection; p.o.: per os; qd: Once every day; q4d: Once every 4 days; qw: Once every week | | | | | |

**Table 12 MDXl74 Groups and Treatment**

| **Group** | **N** | **Treatment** | **Dose (mg/kg)** | **Dosing Route** | **Schedule** |
|---|---|---|---|---|---|
| 1 | 6 | Vehicle | 0 | *i.p.* | qd*5 |
| 2 | 6 | Pemetrexed + Carboplatin | 200+50 | *i.p.* | qd*5 +qw |
| 3 | 6 | Paclitaxel + Carboolatin | 20+50 | *i.p.* | q4d*2 +qw |
| 4 | 6 | Docetaxel +Carboplati | 20+50 | *i.p.* | q4d*2 +qw |
| 5 | 6 | Gemcitabin e+Carbopla | 60+50 | *i.p.* | q4d*2 +qw |
| 6 | 6 | Vinorelbine +Carboplati | 5+50 | *i.p.* | q4d*2+qw |

| | | | | | |
|---|---|---|---|---|---|
| Note: N: mini-PDX device number; i.p.: intraperitoneal injection; p.o.: per os; qd: Once every day; q4d: Once every 4 days; qw: Once every week | | | | | |

**Table 13 MDX186 Groups and Treatment**

| **Group** | **N** | **Treatment** | **Dose (mg/kg)** | **Dosing Route** | **Schedule** |
|---|---|---|---|---|---|
| 1 | 5 | Vehicle | 0 | *i.p.* | qd*5 |
| 2 | 5 | Gemcitabine | 60+5 | *i.p.* | q4d*2 +qw |
| 3 | 5 | Oxaliplatin | 5 | *i.p.* | q4d*2 |
| 4 | 5 | 5-Fu | 25 | *i.p.* | qd*5 |
| 5 | 5 | Nab-Paclitax el | 20 | *i.v.* | qd*5 |
| 6 | 5 | Irinotecan | 50 | *i.p.* | q4d*2 |

| | | | | | |
|---|---|---|---|---|---|
| Note: N: mini-PDX device number; i.p.: intraperitoneal injection; i.v.: intravenous injection; qd: Once every day; q4d: Once every 4 days; qw: Once every week; | | | | | |

**Table 14 MDX189 Groups and Treatment**

| **Group** | **N** | **Treatment** | **Dose (mg/kg)** | **Dosing Route** | **Schedule** |
|---|---|---|---|---|---|
| 1 | 5 | Vehicle | 0 | *i.p.* | qd*5 |
| 2 | 5 | S-1+Oxaliplatin | 10+5 | *p.o. +i.p.* | qd*5+qw |
| 3 | 5 | Docetaxel +Cisplatin+5-Fu | 20+5+5 | *i.p.* | qw+qw+qd *5 |
| 4 | 5 | 5-Fu+Cisplatin | 25+5 | *i.p.* | qd*5+qw |

| | | | | | |
|---|---|---|---|---|---|
| Note: N: mini-PDX device number; i.p.: intraperitoneal injection; p.o.: per os; qd: Once every day; q4d: Once every 4 days; qw: Once every week; | | | | | |

**Table 15 MDX203 Groups and Treatment**

| **Group** | **N** | **Treatment** | **Dose (mg/kg)** | **Dosing Route** | **Schedule** |
|---|---|---|---|---|---|
| 1 | 5 | Vehicle | 0 | *i.p.* | qd*5 |
| 2 | 5 | Gemcitabine | 60+5 | *i.p.* | q4d*2 +qw |
| 3 | 5 | Oxaliplatin | 5 | *i.p.* | q4d*2 |
| 4 | 5 | 5-Fu | 25 | *i.p.* | qd*5 |
| 5 | 5 | Nab-Paclitax el | 20 | *i.v.* | qd*5 |
| 6 | 5 | Irinotecan | 50 | *i.p.* | q4d*2 |

| | | | | | |
|---|---|---|---|---|---|
| Note: N: mini-PDX device number; i.p.: intraperitoneal injection; i.v.: intravenous injection; qd: Once every day; q4d: Once every 4 days; qw: Once every week; | | | | | |

### 6.3 Observation

The protocol and any amendment(s) or procedures involving the care and use of animals in this study were reviewed and approved by the Institutional Animal Care and Use Committee (IACUC) of Shanghai LIDE prior to conduct. During the study, the care and use of animals were conducted in accordance with the regulations of the Association for Assessment and Accreditation of Laboratory Animal Care (AAALAC). After inoculation, the animals were checked daily for morbidity and mortality. At the time of routine monitoring, the animals were checked for any effects of tumor growth and treatments on normal behavior such as mobility, food and water consumption, body weight gain/loss (body weight were measured twice weekly or every other day), eye/hair matting and any other abnormal effect. Death and observed clinical signs were recorded on the basis of the numbers of animals within each subset.

### 6.4 Endpoints

6.4.1 Body weight were measured every day, the treatment periods of these studies were 7 days, at the termination, all mice were euthanized and the Mini-PDX devices were removed for CTG (cell viability) assay. Tumor relative proliferation rate (%) is the indication of the anti-tumor activity.
6.4.2 When V cd7-V do > 0, Tumor relative proliferation rate(%) = (V rnr V do) / (V cdr V do) x100%; When V cd7-V do < 0, Tumor relative proliferation rate(%) = V Td7/ V cd7 x100% (V Td7: the cell viability on day 7 of treatment group; V cd7: the cell viability on day 7 of control group; Vdo: the cell viability on day 0).

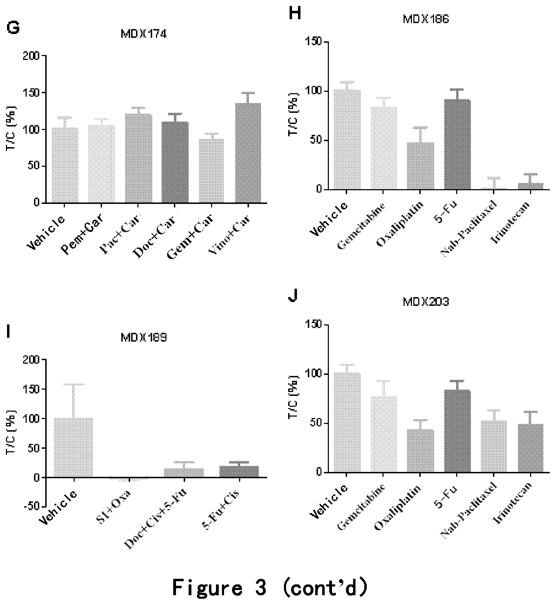

### 7. Results (See Figure 3)

### 8. Summary and Discussion

In MDX133 Mini-PDX models, Paclitaxel treatment (T/C%=52%) could result in a decrease in tumor cell viability, Irinotecan treatment (T/C%=72%), S-1 treatment (T/C%=86%), Oxaliplatin treatment (T/C%=89%) also shown a little anti-tumor activity; 5-Fu treatment (T/C%=103%) shown no anti-tumor activity.

In MDX154 Mini-PDX models, all the treatment group include Gemcitabine treatment (T/C%=100%), Oxaliplatine treatment (T/C%=174%), 5-Fu treatment (T/C%=111%), Nab-paclitaxel treatment (T/C%=162%), Irinotecan treatment (T/C%=227%) shown no anti-tumor activity.

In MDX164 Mini-PDX models, Temozolomide treatment (T/C%=102%) shown no anti-tumor activity.

In MDX165 Mini-PDX models, Temozolomide treatment (T/C%=29%) could result in a decrease in tumor cell viability.

In MDX168 Mini-PDX models, Nab-paclitaxel treatment (T/C%=15%), Irinotecan treatment (T/C%=12%) could result in a decrease in tumor cell viability; Gemcitabine treatment (T/C%=53%), Oxaliplatin treatment (T/C%=75%) also shown a little anti-tumor activity; 5-Fu treatment (T/C%=146%) shown no anti-tumor activity.

In MDX169 Mini-PDX models, Docetaxel and Carboplatin combination treatment (T/C%=20%), Paclitaxel and Carboplatin combination treatment (T/C%=40%) could result in a decrease in tumor cell viability; Pemetrexed and Carboplatin combination treatment (T/C%=55%), Gemcitabine and Carboplatin combination treatment (T/C%=82%), Vinorelbine and Carboplatin combination treatment (T/C%=91 %) also shown a little anti-tumor activity.

In MDXI 74 Mini-PDX models, Gemcitabine and Carboplatin combination treatment (T/C%=86%) shown a little anti-tumor activity; Pemetrexed and Carboplatin combination treatment (T/C%=104%), Paclitaxel and Carboplatin combination treatment (T/C%=120%), Docetaxel and Carboplatin combination treatment (T/C%=110%), Vinorelbine and Carboplatin combination treatment (T/C%=134%) shown no anti-tumor activity.

In MDX186 Mini-PDX models, Nab-paclitaxel treatment (T/C%=0%), Irinotecan treatment (T/C%=5%) could result in a significant decrease in tumor cell viability; Oxaliplatin treatment (T/C%=47%) shown a anti-tumor activity; Gemcitabine treatment (T/C%=83%), 5-Fu treatment (T/C%=90%) also shown a little anti-tumor activity.

In MDX189 Mini-PDX models, S-1 and Oxaliplatin combination treatment (T/C%=-2%), Docetaxel, Cisplatin and 5-Fu combination treatment (T/C%=14% ), 5-Fu and Cisplatin combination treatment (T/C%=19%) could result in a significant decrease in tumor cell viability.

In MDX203 Mini-PDX models, Oxaliplatin treatment (T/C%=43%), Irinotecan treatment (T/C%=48%), Nab-paclitaxel treatment (T/C%=52%) could result in a decrease in tumor cell viability; Gemcitabine treatment (T/C%=77%), 5-Fu treatment (T/C%=83%) also showed a little anti-tumor activity.

### 9. Tumor cell count for samples obtained from surgery and biopsy/needle biopsy tumor samples

Tumor cells numbers are counted using methods known in the art and then converted to tumor cell concentration in the suspension. M: *10⁶/mL.

| | **sample** ID | **Source of sample** | **cancer type** | **tumor cell concentration** |
|---|---|---|---|---|
| 1 | MDX079 | surgical | lung cancer | - |
| 2 | MDX083 | needle biopsy | adenoid cystic carcinoma | 2M |
| 3 | MDX095 | needl e biopsy | peritoneal malignant tnesoshelioma | 1.7M |
| 4 | MDX107 | surgical | Glioblastoma | 0.87M |
| 5 | MDX123 | biopsy | colonrectal cancer | 0.8M |
| 6 | MDX133 | surgical | gastric cancer | 6M |
| 7 | MDX154 | surgical | gallbladder carcinoma | 0.42M |
| 8 | MDX164 | surgical | glioblastoma | 7M |
| 9 | MDX165 | surgical | glioblastoma | 5.9M |
| 10 | MDX168 | surgical | gallbladder carcinoma | 4M |
| 11 | MDX169 | biopsy | lung cancer | 0.64M |
| 12 | MDX174 | biopsy | lung cancer | 0.59M |
| 13 | MDX186 | surgical | pancreatic cancer | 0.5M |
| 14 | MDX189 | surgical | esophagus cancer | 2.1M |
| 15 | MDX203 | surgical | gallbladder carcinoma | 3.2M |

## Claims

1. A method for obtaining an animal model for screening anti-tumor drugs, comprising
(1) cultivating a primary tumor cell obtained from a tumor sample of a patient in a composition comprising:
a) 0.4 mg/L Hydrocortisone;
b) 5 mg/L Insulin;
c) 8.3 µg/L Cholera toxin;
d) 24.2 mg/L Adenine;
e) 10 µg/L EGF;
f) 10 µmol/L Y- 27632;
g) Pen/Strep;
h) 10% FBS;
i) F12 medium
j) DMEM with high glucose
k) Non-Essential Amino Acids Solution;
l) GlutaMAX Supplement; and
m) a mouse embryonic fibroblast cell that has been treated with mitomycin C; and
(2) implanting the primary tumor cell obtained from step (1) into an animal, wherein the animal is a mouse.

2. The method of claim 1, wherein the tumor sample is a surgical sample or a biopsy sample.

3. The method of claim 1, wherein the concentration of the primary tumor cells in the tumor sample obtained from a needle biopsy is less than about 2*10⁶/mL, less than about 1.9*10⁶/mL, less than about 1.8*10⁶/mL, less than about 1.7*10⁶/mL, less than about 1.6*10⁶/mL, less than about 1.5*10⁶/mL, less than about 1.4 *10⁶/mL, less than about 1.3 *10⁶/mL, less than about 1.2 *10⁶/mL, less than about 1.1 *10⁶/mL, less than about 1.0 *10⁶/mL, less than about 0.9 *10⁶/mL, less than about 0.8 *10⁶/mL, less than about 0.7 *10⁶/mL, less than about 0.6 *10⁶/mL, less than about 0.5 *10⁶/mL, less than about 0.4 *10⁶/mL, less than about 0.3 *10⁶/mL, less than about 0.2 *10⁶/mL, less than about 0.1 *10⁶/mL, such as less than about 2 *10⁶/mL, less than about 1.7 *10⁶/mL, less than about 0.8 *10⁶/mL, less than about 0.64 *10⁶/mL, less than about 0.59 *10⁶/mL.

4. The method of claim 1, wherein the mouse is an immunodeficient mouse.

5. The method of claim 1, wherein the primary tumor cell obtained in step (1) is implanted in to the animal in a hollow fiber, in particular the hollow fiber is made of modified polyvinylidene fluoride, more particularly the hollow fiber is made of modified polyvinylidene fluoride and has a cut-off value of 500,000 Dalton.

6. The method of claim 1, wherein the primary tumour cell obtained from step(1) is implanted into a hollow fiber and the hollow fiber is implanted subcutaneously into the mouse.

## Patentansprüche

1. Verfahren zur Gewinnung eines Tiermodells für das Screening von Antitumormitteln, umfassend
(1) das Kultivieren einer aus einer Tumorprobe eines Patienten gewonnenen Primärtumorzelle in einer Zusammensetzung, die umfasst:
a) 0,4 mg/l Hydrocortison;
b) 5 mg/l Insulin;
c) 8,3 µg/l Cholera-Toxin;
d) 24,2 mg/l Adenin;
e) 10 µg/l EGF;
f) 10 µmol/l Y-27632;
g) Pen/Strep;
h) 10 % FBS;
i) F12-Medium
j) DMEM mit hohem Glukosegehalt
k) Lösung nicht essenzieller Aminosäuren;
l) GlutaMAX-Ergänzung; und
m) eine mit Mitomycin C behandelte embryonale Fibroblastenzelle der Maus; und
(2) Implantieren der aus Schritt (1) erhaltenen primären Tumorzelle in ein Tier, wobei das Tier eine Maus ist.

2. Verfahren nach Anspruch 1, wobei die Tumorprobe eine chirurgische Probe oder eine Biopsieprobe ist.

3. Verfahren nach Anspruch 1, wobei die Konzentration der Primärtumorzellen in der aus einer Nadelbiopsie gewonnenen Tumorprobe weniger als etwa 2*106/ml, weniger als etwa 1,9*106/ml, weniger als etwa 1,8*106/ml, weniger als etwa 1,7*106/ml, weniger als etwa 1,6*106/ml, weniger als etwa 1,5*106/ml, weniger als etwa 1,4*106/ml, weniger als etwa 1,3*106/ml, weniger als etwa 1,2*106/ml, weniger als etwa 1,1*106/ml, weniger als etwa 1,0*106/ml, weniger als etwa 0,9*106/ml, weniger als etwa 0,8 *106/ml, weniger als etwa 0,7 *106/ml, weniger als etwa 0,6 *106/ml, weniger als etwa 0,5 *106/ml, weniger als etwa 0,4 *106/ml, weniger als etwa 0,3 *106/ml, weniger als etwa 0,2 *106/ml, weniger als etwa 0,1 *106/ml, beispielsweise weniger als etwa 2 *106/ml, weniger als etwa 1,7 *106/ml, weniger als etwa 0,8 *106/ml, weniger als etwa 0,64 *106/ml, weniger als etwa 0,59 *106/ml.

4. Verfahren nach Anspruch 1, wobei die Maus eine immundefiziente Maus ist.

5. Verfahren nach Anspruch 1, wobei die in Schritt (1) erhaltene primäre Tumorzelle dem Tier in einer Hohlfaser implantiert wird, wobei die Hohlfaser insbesondere aus modifiziertem Polyvinylidenfluorid besteht, genauer gesagt, die Hohlfaser aus modifiziertem Polyvinylidenfluorid besteht und einen Cut-off-Wert von 500.000 Dalton aufweist.

6. Verfahren nach Anspruch 1, wobei die in Schritt (1) erhaltene Primärtumorzelle in eine Hohlfaser implantiert wird und die Hohlfaser subkutan in die Maus implantiert wird.

## Revendications

1. Procédé pour obtenir un modèle animal pour le criblage de médicaments antitumoraux, comprenant
(1) la culture d'une cellule tumorale primaire obtenue à partir d'un échantillon tumoral d'un patient dans une composition comprenant :
a) 0,4 mg/L d'hydrocortisone ;
b) 5 mg/L d'insuline ;
c) 8,3 µg/L de toxine cholérique ;
d) 24,2 mg/L d'adénine ;
e) 10 µg/L d'EGF ;
f) 10 µmol/L de ;
g) Pen/Strep ;
h) 10 % de FBS ;
i) milieu F12
j) DMEM à haute teneur en glucose
k) solution d'acides aminés non essentiels ;
l) supplément GlutaMAX ; et
m) une cellule fibroblastique embryonnaire de souris qui a été traitée avec de la mitomycine C ; et
(2) implanter la cellule tumorale primaire obtenue à l'étape (1) dans un animal, dans lequel l'animal est une souris.

2. Procédé selon la revendication 1, dans lequel l'échantillon tumoral est un échantillon chirurgical ou un échantillon de biopsie.

3. Procédé selon la revendication 1, dans lequel la concentration des cellules tumorales primaires dans l'échantillon tumoral obtenu à partir d'une biopsie à l'aiguille est inférieure à environ 2*106/mL, inférieure à environ 1,9*106/mL, inférieure à environ 1,8*106/mL, inférieure à environ 1,7*106/mL, inférieure à environ 1,6*106/mL, inférieure à environ 1,5*106/mL, inférieure à environ 1,4 *106/mL, inférieure à environ 1,3 *106/mL, inférieure à environ 1,2 *106/mL, inférieure à environ 1,1 *106/mL, inférieure à environ 1,0 *106/mL, inférieure à environ 0,9 *106/mL, moins d'environ 0,8 *106/mL, moins d'environ 0,7 *106/mL, moins d'environ 0,6 *106/mL, moins d'environ 0,5 *106/mL, moins d'environ 0,4 *106/mL, moins d'environ 0,3 *106/mL, moins d'environ 0,2 *106/mL, moins d'environ 0,1 *106/mL, tel que moins d'environ 2 *106/mL, moins d'environ 1,7 *106/mL, moins d'environ 0,8 *106/mL, moins d'environ 0,64 *106/mL, moins d'environ 0,59 *106/mL.

4. Procédé selon la revendication 1, dans lequel la souris est une souris immunodéficiente.

5. Procédé selon la revendication 1, dans lequel la cellule tumorale primaire obtenue à l'étape (1) est implantée dans l'animal dans une fibre creuse, en particulier la fibre creuse est constituée de polyfluorure de vinylidène modifié, plus particulièrement la fibre creuse est constituée de polyfluorure de vinylidène modifié et a une valeur de coupure de 500 000 daltons.

6. Procédé selon la revendication 1, dans lequel la cellule tumorale primaire obtenue à l'étape (1) est implantée dans une fibre creuse et la fibre creuse est implantée par voie sous-cutanée dans la souris.
